# EUROPEAN PATENT SPECIFICATION

(11) **EP 3 890 693 B1**
(45) Date of publication and mention of the grant of the patent: **16.04.2025**
(21) Application number: 19813330.8
(22) Date of filing: 03.12.2019
(51) Int. Cl.: A61K 8/34, A61K 8/41, A61K 8/44, A61K 8/46, A61Q 5/04, A61K 8/60, A61K 8/04, A61K 8/19

(54) **PROCESS FOR RESHAPING KERATIN FIBERS**
VERFAHREN ZUR UMFORMUNG VON KERATINFASERN
PROCÉDÉ DE REMODELAGE DE FIBRES DE KÉRATINE

(30) Priority: 06.12.2018 EP 18210802
(43) Date of publication of application: 13.10.2021
(73) Proprietor: Kao Germany GmbH, 64297 Darmstadt Hessen (DE)
(72) Inventor: NÖCKER, Bernd, 64297 Darmstadt (DE); BREAKSPEAR, Steven, 64297 Darmstadt (DE); JIAN, Niu, 64297 Darmstadt (DE)
(74) Representative: Miller, Tobias
(86) International application number: PCT/EP2019/083515
(87) International publication number: WO 2020/115053

(56) References cited:
- WO-A1-2011/155076
- WO-A1-2015/068728
- WO-A1-2019/074128
- WO-A2-2015/001074

## Description

### Field of the invention

The present invention relates to a process for reshaping keratin fibers, a foam dispenser comprising a reshaping composition, and a kit-of-parts comprising a reshaping composition and a moisture barrier.

### Background of the invention

Current permanent reshaping processes require steps of reducing the hair and later oxidizing the hair. The reducing step employs reducing agents, such as thioglycolic acid, in order to cleave disulphide bonds within the hair and to allow protein chain movement. In a subsequent step the bonds are reformed by addition of an oxidizing composition, typically comprising hydrogen peroxide. In Western countries these well known processes include cold perming and hot perming techniques, whereas, especially in Asian countries, a digital perming as a specialty of the hot perming process is preferred. For a digital perming process the temperature of the perm is controlled by an electronic device, often being equipped with a microprocessor.

These multi-step processes are time-consuming (up to 3 hours for a cold perm/5 hours for a digital perm), require a high level of winding skill from the stylist, cause hair damage and, at worst, can lead to hair loss due to over-processing. Over-processing typically stems from either leaving the reducing agent for too long on the hair, or heating the hair to inappropriate temperatures for a too long time. To avoid over-processing, a test curler is typically carried out on a streak of the clients hair prior to the full process being performed, in order to determine the optimum processing time; this adds further to the time needed and requires further skills/education from the stylist. Apart from all the process challenges, the reducing composition has a strong and acrid odour disliked by stylists and consumers alike. In contrast to the common reducing techniques, EP2512423 discloses a perming process and composition which do not make use of reducing or oxidizing agents, but of compositions comprising surfactants at a pH below 7.

WO 2011/155076 A1 discloses a process for treating keratin fibers under mechanical tension to be heated in an occlusive space, with a composition which is free of reducing agents and comprises one or several alkaline agents. This process is said to achieve better perm performance without generating significant or critical degradation. The process may be performed without any step of oxidizing the keratin fibers.

Despite all efforts of the prior art and the long experience with reducing/oxidizing processes, there is a real need for perming processes which do not show the disadvantages as presented above, and additionally possess an improved handling, spread ability, and convenient use with minimal risk of errors from the user.

### Summary of the invention

Therefore, the first object of the present invention is a process for reshaping keratin fibers, preferably human keratin fibers, more preferably human hair characterized in that it comprises the steps of:
a) putting keratin fibers under mechanical tension,
b) applying to keratin fibers a non-reducing, non-oxidizing alkaline composition with a pH in the range of 7 to 12, preferably 8 to 11, comprising
   i) one or more alkalizing agent(s),
   ii) one or more foaming surfactant(s) selected from non-ionic and/or anionic and/or zwitterionic and/or amphoteric surfactant(s), and/or their mixtures,
c) covering the keratin fibers with a moisture barrier,
d) heating the keratin fibers to a temperature in the range of 50°C to 230°C,
e) removing the moisture barrier from keratin fibers,
f) releasing tension from keratin fibers,
g) optionally rinsing-off the keratin fibers,
wherein process steps a), b), and f), g) can be executed in any order.

The second object of the present invention is a kit-of-parts comprising in a separately packed container a composition as defined for step b) as defined above, and a moisture barrier as defined for step c).

The PCT-application WO 2019/074128 (L'Oréal), filed on 05.10.2018 and published on 18 April 2019, is prior art according to Art. 153(5) EPC in connection with Art. 54(3) EPC. The processes and kits-of-parts disclosed in this earlier application, which overlapped with present application, have been excluded from the scope of the claims.

The third object of the present invention is a foam dispenser comprising a non-reducing, non-oxidizing alkaline composition having a pH in the range of 7 to 12, preferably 8 to 11 comprising:
i) one or more alkalizing agent(s),
ii) one or more foaming surfactant(s) selected from non-ionic and/or anionic and/or zwitterionic and/or amphoteric surfactant(s), and/or their mixtures, and
iii) one or more propellant(s),
wherein the total concentration of propellants is in the range of 30% to 90% by weight, preferably in the range of 45% to 85% by weight, more preferably in the range of 60% to 80% by weight, calculated to the total of the composition.

### Detailed description of the invention

It has been surprisingly found by the inventors of the present invention that a composition having a pH in the range of 7 to 12 comprising surfactants used in a perming process in combination with a moisture barrier delivered superior, long-lasting curling results having increased waving properties, the process having minimal risk of over-processing and conferring low damage of the hair fibers.

### Non-reducing, non-oxidizing alkaline composition of step b)

The term 'non-reducing' within the meaning of the present invention is to be understood that the composition of step b) is free of agents causing a reduction of disulfide bonds in the keratin fibers. The term does not exclude low amounts of reducing agents which may be necessary in the composition for stabilizing purposes.

The term 'non-oxidizing' within the meaning of the present invention is to be understood that the composition of step b) is free of oxidizing agents causing an oxidation of disulfide bonds in the keratin fibers. The term does not exclude low amounts of oxidizing agents which may be necessary in the composition for stabilizing purposes.

The composition comprises one or more alkalizing agent(s) to yield a pH in the range of 7 to 12. The preferred pH range is 8 to 11 from the viewpoint of achieving a good curling result in combination with low hair damage.

Suitable alkalizing agents are selected from ammonia and/or its salts and/or guanidine and/or its salt(s) and/or organic alkyl and/or alkanol amines according to the general structure 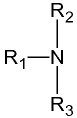 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and/or their salts, and/or their mixtures.

The organic alkyl and/or alkanol amine according to the structure above may be selected from mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine, tris-(hydroxymethyl)-aminomethane, and/or aminomethyl propanol. Equally suitable are salts of alkyl and/or alkanol amines with a counterion preferably selected from chloride and/or hydrogen chloride, nitrate, sulphate, phosphate, hydrogenphosphate, dihydrogenphosphate, citrate, acetate, sulphite, benzoate, salicylate.

In one aspect of the present invention, the alkalizing agent of the alkaline composition of step b) is selected from ammonia and/or its salt(s) and/or aminomethyl propanol and/or monoethanolamine, and/or diethanolamine, and/or tris-(hydroxymethyl)-aminomethane, and/or their salts.

In another aspect of the present invention, the alkalizing agent of the alkaline composition of step b) is ammonia and/or its salts.

In a most preferred aspect of the present invention the alkalizing agent is a mixture of one or more alkyl amine(s) and/or their salt(s) with ammonia and/o its salt(s). A preferred combination is, for example, monoethanolamine and/or its salt(s) and ammonia and/or its salt(s). Another preferred combination is aminomethyl propanol and/or its salt(s) and ammonia and/or its salt(s). The aforementioned combinations are preferred from the viewpoint of reducing ammonia smell while maintaining good curling performance.

The preferred lower total concentration of alkalizing agent(s) in the alkaline composition of step b) preferably is 0.1% by weight or more, more preferably is 0.25% by weight or more, further more preferably is 0.5% by weight or more, from the viewpoint of achieving sufficient alkalinity and good curling results.

The preferred upper total concentration of alkalizing agent(s) in the alkaline composition of step b) preferably is 10% by weight or less, more preferably is 7.5% by weight or less, further more preferably is 5% by weight or less, form the viewpoint of reducing hair damage.

For attaining the above mentioned effects, the preferred total concentration of alkalizing agent(s) in the alkaline composition of step b) is in the range of 0.1% to 10% by weight, preferably in the range of 0.25% to 7.5% by weight, more preferably in the range of 0.5%to 5% by weight, calculated to the total of the composition of step b).

### Foaming surfactants of the composition of step b)

The non-reducing, non-oxidizing composition of step b) comprises one or more foaming surfactant(s) selected from non-ionic and/or anionic and/or zwitterionic and/or amphoteric surfactant(s), and/or their mixtures.

### Anionic surfactants

Preferably, the anionic surfactants of the alkaline composition of step b) are selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures.

Suitable alkyl sulfate or preferably ethoxylated alkyl ether sulfate surfactant or mixtures thereof have an alkyl chain length of C₁₀ to C₂₂.

Suitable example foaming surfactants are laureth sulfates, coceth sulfate, pareth sulfate, capryleth sulphate, myreth sulfate, oleth sulfate, deceth sulfate, trideceth sulfate, coco sulphate, C₁₀-C₁₆ alkyl sulphate, C₁₁-C₁₅ alkyl sulphate, C₁₂-C₁₈ alkyl sulphate, C₁₂-C₁₅ alkyl sulphate, C₁₂-C₁₆ alkyl sulphate, C₁₂-C₁₃ alkyl sulfate, lauryl sulphate, myrystyl sulphate, palm kernel sulphate, cetearyl sulfate, cetyl sulphate, decyl sulphate, oleyl sulphate, behenyl sulphate and/or their salts. All of the aforementioned anionic surfactants may or may not be ethoxylated at various degrees.

Cations for the surfactants may be selected from sodium, potassium, magnesium and/or ammonium.

Further suitable anionic surfactants are alkyl ether carboxylates derived from alkanols having 6 to 22 carbon atoms, preferably one satisfying the following formula:

R₆₀-O-(CH₂CH₂O)ₙ₁₅-CH₂COO- M+

wherein R₆₀ is an alkyl residue having 6 to 22 carbon atoms, n has a value in the range of 1 to 15, preferably 2 to 10, more preferably 2.5 to 7, and M+ is an appropriate cation, selected from the group consisting of an alkali metal, an alkaline earth metal, ammonium, an alkylammonium, an alkanolammonium or a glucammonium.

Particularly preferred are compounds of the above formula wherein R60 is an alkyl residue having 12 to 14 carbon atoms; n has a value in the range of 2.5 to 5 and M+ is an alkali metal cation such as sodium or potassium. Alkyl ether carboxylates are preferably used as liquid diluted aqueous solutions having a solid content lower than 30% by weight.

Alkyl ether carboxylates are obtained by ethoxylation and subsequent carboxymethylation of fatty alcohols.

Examples of commercially available alkyl ether carboxylate acid salts are marketed under the trade name AKYPO^{®} by Kao Chemicals GmbH.

The most preferred anionic foaming surfactant is sodium lauryl sulfate.

### Non-ionic surfactants

Suitable non-ionic surfactants of the alkaline composition of step b) are selected from alkyl polyglycosides, ethoxylated triglycerides, ethoxylated fatty alcohols, ethoxylated fatty acid esters, and/or their mixtures.

Suitable nonionic surfactants are alkyl polyglycosides according to the general structure:

R₂₃O(R₂₄O)ₜZₓ

Wherein Z denotes a carbohydrate with C₅ to C₆, R₂₃ is an alkyl group with C₈ to C₁₈, R₂₄ is methyl, ethyl or propyl, t ranges from 0 to 10, and x ranges from 1 to 5. Suitable compounds according to this structure are C₉-C₁₁ alkylpolyglycoside, the structures disclosed in EP-A 70 074, and JP 2015-123019A.

The preferred compounds according to the structure of above are decyl glucoside, lauryl glucoside, and coco glucoside, and the most preferred one is decyl glucoside.

Further suitable examples for non-ionic surfactants are N-alkylpolyhydroxyalkylamide type surfactants according to the following general formula: 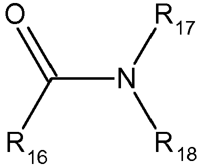 wherein R₁₆ is a linear or branched, saturated or unsaturated alkyl chain with C₁₁ to C₂₁, R₁₇ is linear or branched alkyl, or linear or branched hydroxyalkyl with C₁ to C₄, and R₁₈ is a linear or branched polyhydroxyalkyl chain with C₃ to C₁₂ and 3 to 10 hydroxyl groups.

Such compounds are disclosed in cosmetic compositions in WO96/27366 and their synthesis is disclosed in US1985424, US2016962, US2703798, and WO92/06984.

The preferred N-alkylpolyhydroxyalkylamide type surfactants have the following structure: 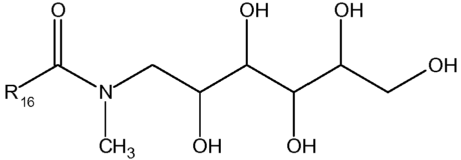 where R₁₆ has the same denotation as above for the general structure of N-alkylpolyhydroxyalkylamide type surfactants. The preferred surfactants as displayed above are known as N-methyl-N-acylglucamides.

The most preferred N-alkylpolyhydroxyalkylamide type surfactants are selected from lauroyl/myristoyl methyl glucamide and coco methyl glucamide.

Further suitable examples for non-ionic surfactants are ethoxylated fatty alcohol of the following general structure

R₂₅ (OCH₂CH₂)ₙ₄ OH

wherein R₂₅ is straight or branched, saturated or unsaturated alkyl chain which may be synthetic or natural with a C chain length in the range of 8 to 40, preferably 9 to 30 and more preferably 9 to 24 and n4 is a number in the range of 5 to 40, preferably 9 to 30.

Non-limiting suitable examples of the fatty alcohol ethoxylates are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be comprised in the compositions as a mixture of more than one surfactant.

Further suitable nonionic surfactants are polypropylene glycol ethers of fatty alcohol according to general structure

R₂₅ (OCH₂-CH₂-CH₂)ₙ₅ OH

wherein R₂₅ is straight or branched, saturated or unsaturated fatty alcohol which may be synthetic or natural with a C chain length in the range of 8 to 40, preferably 9 to 30 and more preferably 9 to 24 and n5 is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

R₂₆ C(O) (OCH₂CH₂)ₙ₆ OH

wherein R₂₆ is straight or branched, saturated or unsaturated alkyl group which may be synthetic or natural with a C chain length in the range of 7 to 39, preferably 9 to 29 and more preferably 9 to 23 and n6 is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate,

PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15 Stearate, PEG-20 Stearate, PEG-25 Stearate, PEG-30 Stearate, PEG-35 Stearate and PEG-40 Stearate.

Further suitable nonionic surfactants are propoxylated fatty acid esters of the following general structure

R₂₇ C(O) (OCH₂-CH₂-CH₂)ₙ₈ OH

wherein R₂₇ is straight or branched, saturated or unsaturated alkyl group which may be synthetic or natural with a C chain length in the range of 7 to 39, preferably 9 to 29 and more preferably 9 to 23 and n8 is a number in the range of 1 to 40, preferably 9 to 30.

Suitable non-limiting examples are PPG-15 Isostearate, PPG-9 Laurate, PPG-26 Oleate and PPG-36 Oleate.

Further suitable nonionic surfactants are ethoxylated and propoxylated fatty alcohols of the following general structure

R₂₈ (OCH₂-CH₂-CH₂)ₙ₉ (OCH₂CH₂)ₙ₁₀ OH

wherein R₂₈ is straight or branched, saturated or unsaturated alkyl group which may be synthetic or natural with a C chain length in the range of 7 to 39, preferably 9 to 29 and more preferably 9 to 23 and n9 and n10 may be the same or different and are a number in the range of 1 to 40.

Further suitable nonionic surfactants are ethoxylated triglycerides. Well-known and commonly used examples are ethoxylated castor oil such as PEG-40 hydrogenated castor oil or and PEG-60 hydrogenated castor oil.

The preferred non-ionic surfactant(s) are selected from alkyl polyglycoside(s), ethoxylated and/or propoxylated fatty alcohols, ethoxylated and/or propoxylated triglycerides, ethoxylated and/or propoxylated fatty alcohols, ethoxylated or propoxylated fatty acid esters, N-alkylpolyhydroxyalkylamides, preferably they are selected from C₈-C₂₂ alkyl polyglycoside(s), more preferably they are selected from decyl glucoside, lauryl glucoside, and coco glucoside, and further more preferably it is coco glucoside.

### Amphoteric/zwitterionic surfactants

Suitable amphoteric/zwitterionic surfactants may be selected from compounds according to the general structure(s) 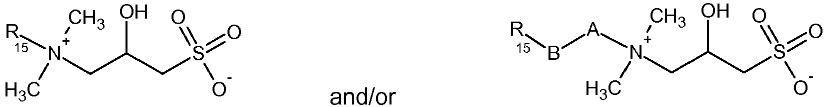 wherein R₁₅ is a straight or branched, saturated or unsaturated, substituted or unsubstituted alkyl chain with a carbon number of C₁₀ to C₂₂, preferably R₁₅ is a straight alkyl chain with a carbon number of C₁₀ to C₁₆, A is a straight alkyl chain with a carbon number of C₁ to C₆ or a branched alkyl chain with a carbon number of C₃ to C₆, preferably A is a linear alkyl chain with a carbon number of C₃, and B is an amide or an ester group.

Suitable compounds are known as hydroxysultaine surfactants, such as cocoamidopropyl hydroxysultaine, laurylamidopropyl hydroxysultaine, erucamidopropyl hydroxysultaine, lauryl hydroxysultaine, and cocoyl hydroxysultaine, and/or their salt(s).

Further suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

The preferred amphoteric/zwitterionic surfactant(s) is/are selected from alkylamido betaines and/or alkylamidoalkyl betaine surfactants.

### Surfactant concentration

The total lower concentration of surfactants in the alkaline composition of step b) is in the range of 0.1% by weight or more, preferably 0.5% by weight or more, more preferably 0.75% by weight or more, calculated to the total of the composition of step b), form the viewpoint of good wettability of the keratin fibers.

The total upper concentration of surfactants in the alkaline composition of step b) is in the range of 10% by weight or less, preferably 8% by weight or less, more preferably 5% by weight or less, calculated to the total of the composition of step b), from the viewpoint of generating a foam, which may be dispensable from a foam dispenser.

For attaining the above-mentioned effects, the total concentration of surfactants in the alkaline composition of step b) is in the range of 0.1% to 10% by weight, preferably 0.5% to 8% by weight, more preferably 0.75% to 5% by weight, calculated to the total of the composition of step b).

### Foam booster for composition of step b)

The composition of step b) may further comprise a foam booster, preferably a compound according to the general structure 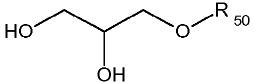 wherein R50 is a linear or branched alkyl chain with a total carbon number of C3 to C12, preferably C3 to C8, more preferably R50 is a branched alkyl chain with a total carbon number of C8, preferably it is ethylhexyl glycerin.

Suitable compounds are propyl glycerine, butyl glycerine, pentyl glycerine, hexyl glycerine, heptyl glycerine, octyl glycerine, nonyl glycerine, decyl glycerine, undecyl glycerine, dodecyl glycrin, ethylhexyl glycerine.

The preferred compound is ethylhexyl glycerine.

Total concentration of the foam booster in the alkaline composition of step b) in the range of 0.1% to 1% by weight, preferably from 0.2% to 1% by weight, more preferably from 0.25% to 1% by weight, calculated to the total of the composition of step b).

### Viscosity of the composition

The viscosity of the composition preferably is suitable for dispensing with a foam dispenser. Therefore, it may have a viscosity in the range of 1 to 2,000 mPas, preferably 10 to 1,500 mPas, more preferably 20 to 1,000 mPas.

The viscosity of the composition may be adjusted by all means available to the skilled person. Such means include the addition of a thickening polymer, preferably an acrylate-based thickening polymer, addition of organic solvents, or of highly viscous compounds such as fatty alcohols, oils such as vegetable oil or silicone oils.

The skilled person may also make use of all of the aforementioned options to adjust the viscosity of the composition.

### Moisture barrier of step c)

Preferably the moisture barrier of step c) is a foil or wrap impermeable for water vapor, or housing made of a material impermeable for water vapor, with the provision that the selected materials for the moisture barrier are heat resistant up to the selected process temperature.

In principle, many materials are suitable for serving as moisture barrier such as aluminum foil, plastic foil, and/or plastic device which enclose the curler and/or hair streak. Alternatively, certain types of anti-flammable fabric is equally suitable. The purpose of the moisture barrier is to keep the hair moist over the total processing time of heating.

Suitable examples are re-sealable zipper storage bags made of materials such a slow density polyethylene.

### Winding and heating process

It is preferred form the viewpoint of convenience that for process step a) the hair is put under mechanical tension on a curler or roller having heating means. After completion of step d) the curler may then be connected to a digital perm machine for heating the curler and the hair.

It is preferred from the viewpoint of minimizing hair damage that the hair is heating in step d) in the range of 80°C to 180°C, preferably 85°C to 140°C, more preferably 90°C to 120°C.

From the viewpoint of having a short processing time and a speedy hair treatment, the heating time of step d) preferably is in the range of 2 min to 45 min, preferably in the range of 5 min to 30 min, more preferably in the range of 5 min to 20 min.

### Foam dispenser

Suitable foam dispensers are non-aerosol foam dispenser as well as aerosol foam dispenser for dispensing the composition of step b).

The non-aerosol foam dispensers usable in the invention are, for example, known pump foam dispensers having a foam discharging unit, squeeze foam dispensers, electric beaters, and accumulator type pump foam dispensers. More specific examples include "Pump Foamer E3 type" and "Pump Foamer F2 type" [each, product of Daiwa Can, Food & Packaging (vol.35, No.10, p588 to 593(1994); vol.35, No.11, p624 to 627(1994); vol.36, No.3, p154 to 158(1995))], "S1 squeeze foamer" (product of Daiwa Can, JP-A-7-215352), an electric beater (product of Panasonic Electric Works), and an air spray foamer (product of Airspray International). As the foam dispenser for use of the present invention dispenses a composition with high alkalinity, a pump foam dispenser and a squeeze foam dispenser are preferred because they are inexpensive, convenient, and resistant to alkaline compositions.

The pump foam dispenser or squeeze foam dispenser have a foam production portion such as net. It has preferably a thin net because when the composition dries into a solid and causes clogging, the flow of foams upon subsequent discharging time can immediately dissolve the solid and eliminate clogging. In this case, the net has preferably a mesh size of from 50 to 280 mesh, more preferably from 90 to 250 mesh, even more preferably from 130 to 220 mesh. The term "mesh" as used herein means the number of openings per inch. Use of a net with a mesh falling within the above range enables creamy foams. Examples of the material of such a net include nylon, polyethylene, polypropylene, polyester, Teflon, carbon fiber, and stainless steel. Of these, nylon, polyethylene, polypropylene, and polyester are more preferred, with nylon being even more preferred.

The non-aerosol dispenser to be used for the purpose of the present invention is equipped with at least one net, preferably a plurality of such nets. From the standpoint of economy and stability of foams, it is preferred to place the net at two places, that is, the mixing chamber and the top portion of the foam dispenser.

Another aspect of the present invention is dispensing the composition of step b) from an aerosol dispenser. Aerosol dispenser require the composition to be filled into an aerosol can, closing the can, and then filling the can with a cosmetically acceptable propellant.

Suitable propellants are carbon dioxide, dimethylether, n-butane, iso-butane, n-pentane, n-hexane, liquefied petroleum gas, hydrofluorcarbon gases, and/or their mixtures.

The composition may then comprise propellants at a total concentration of 30% to 90% by weight, preferably 45% to 85% by weight, more preferably 60% to 80% by weight, calculated to the total of the composition of step b).

### Optional Ingredients of the composition of step b)

The non-reducing, non-oxidizing alkaline composition of step b) may further comprise cationic polymers, amino acids, UV filters, any type of hair dyes.

Particularly preferred are cationic polymers such as the ones known under their CTFA name Polyquaternium, for example Polyquaternium 6, Polyquaternium 10, Polyquaternium 16, and Polyquaternium 37.

Preferably, the concentration of cationic polymers in the composition is in the range of 0.01% by weight to 1% by weight, calculated to the total weight of the composition.

The following examples are to illustrate the present invention, but not to limit it.

### EXAMPLES

### Example 1

The following compositions were prepared by conventional formulation and mixing techniques:

| Ingredient | Comparative comp. [% by weight] | Inventive comp. 1 [% by weight] | Inventive comp. 2 [% by weight] | Inventive comp. 3 [% by weight] |
|---|---|---|---|---|
| Ammonia solution (25%) | 10.0 | 10.0 | 10.0 | 10.0 |
| Fragrance | 0.5 | 0.5 | 0.5 | 0.5 |
| Cetrimonium chloride | 1.0 | - | - | - |
| Sodium lauryl sulfate | - | 1.0 | - | - |
| Coco-glucoside | - | - | 1.0 | - |
| Cocoamido-propylbetaine | - | - | - | 1.0 |
| Water | Ad 100.0 | | | |

The pH of the compositions was adjusted to 9.8 by suitable acids/bases.

The viscosities of the compositions were measured with a Brookfield viscometer and spindle #6. They were found to be in the range of 20 - 100 mPas for the compositions above.

Human hair streaks (Caucasian, 21 cm long, 2 g per bundle) were purchased from Fischbach + Miller Haar, Laupheim, Germany. The hair streaks were shampooed with a commercially available shampoo under the brand name Goldwell Deep Cleansing Shampoo. Then the streaks were towel dried. 1 g of the compositions was dispensed and applied to the hair streaks. The streaks were then winded on perming rods possessing an electrical heating system. Prior to heating, each of the rods were covered with a plastic bag (commercial re-sealable zipper storage bag) made of low-density polyethylene. The rods were then heated to a temperature in the range of 90°C to 110°C for 20 min with a digital perming machine. Then the rods were allowed to cool down, the plastic bag was removed, and the hair was shampooed with the same shampoo from above. The streaks were then blow-dried.

Assessment of curling efficiency was investigated by measuring the mean curl diameters of individual fibers from the shaped tresses and immersing them in water in a petri dish. After 5 minutes immersion, the fibers were swirled with a finger to allow them to adopt a curled shape. The diameter of the curl was then measured and this repeated for five fibers per treatment. The table below reports the experimental results:

| **Parameter** | **Untreated hair streak** | **Comp. comp.** | **Inventive comp. 1** | **Inventive comp. 2** | **Inventive comp. 3** |
|---|---|---|---|---|---|
| **Mean curl diameter [mm]** | 59 | 33 | 26 | 29 | 27 |
| **SD curl diameter [mm]** | 2.1 | 1.8 | 1.3 | 0.8 | 1.1 |

As a result it was found that the three inventive compositions could delivered superior curling results and better waviness.

The following examples are within the scope of the present invention.

### EXAMPLE 2

| | **% by weight** |
|---|---|
| Ammonia solution (25%) | 2.5 |
| Monoethanolamine | 2.0 |
| 1,2-propandiol | 10.0 |
| Polyquaternium 10 | 0.5 |
| Sodium laureth sulfate (1-5 EO) | 2.0 |
| Water | ad 100.0 |

The pH of the composition is adjusted to 9.0 by suitable acids/bases.

### Example 3

| | **% by weight** |
|---|---|
| Ammonia solution (25%) | 2.5 |
| 1,2-propandiol | 10.0 |
| Polyquaternium 16 | 0.5 |
| Coco glucoside | 2.0 |
| Water | ad 100.0 |

The pH of the composition is adjusted to 9.5 by suitable acids/bases.

The composition from above is filled into an aerosol can. The can is closed and dimethylether is added as a propellant (composition/dimethylether 70/30). The composition is then dispensed from the aerosol can.

### Example 4

| | **% by weight** |
|---|---|
| Guanidin | 1.5 |
| Cocoyl betaine | 1.0 |
| Ammonia solution (25%) | ad pH 10.0 |
| Acrylates copolymer | q.s. to yield a viscosity of 1,800 mPas |
| Water | ad 100.0 |

The pH of the composition is adjusted to 8.5 by suitable acids/bases.

The composition is filled into and dispensed from a squeeze foamer.

## Claims

1. A process for reshaping keratin fibers, preferably human keratin fibers, more preferably human hair **characterized in that** it comprises the steps of:
a) putting keratin fibers under mechanical tension,
b) applying to keratin fibers a non-reducing, non-oxidizing alkaline composition with a pH in the range of 7 to 12, preferably 8 to 11, comprising
i) one or more alkalizing agent(s),
ii) one or more foaming surfactant(s) selected from non-ionic and/or anionic and/or zwitterionic and/or amphoteric surfactant(s), and/or their mixtures,
c) covering the keratin fibers with a moisture barrier,
d) heating the keratin fibers to a temperature in the range of 50°C to 230°C,
e) removing the moisture barrier from keratin fibers,
f) releasing tension from keratin fibers,
g) optionally rinsing-off the keratin fibers,
wherein process steps a), b), and f), g) can be executed in any order, and
wherein the following non-reducing- non-oxidizing compositions are excluded from step b):
| | Ex. 3 | Ex. 4 | Ex. 5 | Comp. Ex. 5 |
|---|---|---|---|---|
| NaOH | 2.4 | 2.4 | 2.4 | 2.4 |
| Monoethanolamine | 3.6 | 3.6 | 3.6 | 3.6 |
| Taurine | 8.7 | 8.7 | 8.7 | 8.7 |
| Magnesium Gluconate | 0.125 | 0.125 | 0.125 | 0.125 |
| Pentylene Glycol | 10 | 7.5 | 5 | - |
| Ethanol | - | 2.5 | 5 | - |
| Hydroxyethylcellulose | 1.5 | 1.5 | 1.5 | 1.5 |
| Polyquaternium-67 | 0.5 | 0.5 | 0.5 | 0.5 |
| Dimethicone | 0.2 | 0.2 | 0.2 | 0.2 |
| Mineral Oil | 5 | 5 | 5 | 5 |
| Steareth-2 | 2 | 2 | 2 | 2 |
| Steareth-20 | 2 | 2 | 2 | 2 |
| Water | qsp 100 | qsp 100 | qsp 100 | qsp 100 |
| pH | 10.7 | 10.7 | 10.7 | 10.7 |

2. The process according to the claim 1 **characterized in that** alkalizing agent of the alkaline composition of step b) is selected from ammonia and/or its salts and/or guanidine and/or its salt(s) and/or organic alkyl and/or alkanol amines according to the general structure 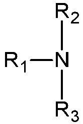 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and/or their salts, and/or their mixtures.

3. The process according to claim 1 and/or 2 **characterized in that** the alkalizing agent of the alkaline composition of step b) is selected from aminomethyl propanol and/or monoethanolamine, and/or tris-(hydroxymethyl)-aminomethane, and/or diethanolamine, and/or their salts.

4. The process according to any of the preceding claims **characterized in that** the alkalizing agent of the alkaline composition of step b) is ammonia and/or its salts.

5. The process according to any of the preceding claims **characterized in that** the total concentration of alkalizing agent of the alkaline composition of step b) is in the range of 0.1% to 10% by weight, preferably in the range of 0.25% to 7.5% by weight, more preferably in the range of 0.5%to 5% by weight, calculated to the total weight of the composition of step b).

6. The process according to any of the preceding claims **characterized in that** the anionic surfactants of the alkaline composition of step b) are selected from ethoxylated or non-ethoxylated alkyl ether sulfate surfactants, ethoxylated or non-ethoxylated alkyl sulfates, ethoxylated and/or non-ethoxylated alkyl ether carboxylates, and/or their mixtures.

7. The process according to any of the preceding claims **characterized in that** the non-ionic surfactants of the alkaline composition of step b) are selected from alkyl polyglycoside(s), ethoxylated and/or propoxylated fatty alcohols, ethoxylated and/or propoxylated triglycerides, ethoxylated and/or propoxylated fatty alcohols, ethoxylated or propoxylated fatty acid esters, N-alkylpolyhydroxyalkylamides, and/or their mixtures.

8. The process according to any of the preceding claims **characterized in that** the total concentration of surfactants in the alkaline composition of step b) is in the range of 0.1% to 10% by weight, preferably 0.5% to 8% by weight, more preferably 0.75% to 5% by weight, calculated to the total of the composition of step b).

9. The process according to any of the preceding claims **characterized in that** the alkaline composition of step b) comprises a compound according to the general structure 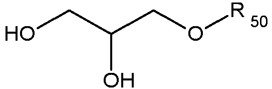 wherein R50 is a linear or branched alkyl chain with a total carbon number of C3 to C12, preferably C3 to C8, more preferably R50 is a branched alkyl chain with a total carbon number of C8, preferably it is ethylhexyl glycerin.

10. The process according to any of the preceding claims **characterized in that** keratin fibers are heated according to step d) in the range of 80°C to 180°C, preferably 85°C to 140°C, more preferably 90°C to 120°C.

11. The process according to any of the preceding claims **characterized in that** the heating time of step d) is in the range of in the range of 2 min to 45 min, preferably in the range of 5 min to 30 min, more preferably in the range of 5 min to 20 min.

12. The process according to any of the preceding claims **characterized in that** for process step a) the hair is put under mechanical tension on a curler or roller having heating means.

13. The process according to any of the preceding claims **characterized in that** the moisture barrier of step c) is a foil or wrap impermeable for water vapor, or housing made of a material impermeable for water vapor, with the provision that the selected materials for the moisture barrier are heat resistant up to the selected process temperature.

14. Kit-of-parts comprising in a separately packed container a composition as defined for step b) in the claims 1 to 9, and a moisture barrier as defined in claim 13 for step b).

15. A foam dispenser comprising a non-reducing, non-oxidizing alkaline composition having a pH in the range of 7 to 12, preferably 8 to 11 comprising:
i) one or more alkalizing agent(s),
ii) one or more foaming surfactant(s) selected from non-ionic and/or anionic and/or zwitterionic and/or amphoteric surfactant(s), and/or their mixtures, and
iii) one or more propellant(s):
wherein the total concentration of propellants is in the range of 30% to 90% by weight, preferably in the range of 45% to 85% by weight, more preferably in the range of 60% to 80% by weight, calculated to the total of the composition.

## Patentansprüche

1. Verfahren zur Umformung von Keratinfasern, vorzugsweise menschlichen Keratinfasern, insbesondere menschlichem Haar, **dadurch gekennzeichnet, dass** es die folgenden Schritte umfasst:
a) Keratinfasern unter mechanische Spannung setzen,
b) Auftragen einer nicht reduzierenden, nicht oxidierenden alkalischen Zusammensetzung mit einem pH-Wert im Bereich von 7 bis 12, vorzugsweise 8 bis 11, auf Keratinfasern, umfassend
i) einem oder mehreren Alkalisierungsmittel(n),
ii) ein oder mehrere schäumende(s) Tensid(e), ausgewählt aus nichtionischen und/oder anionischen und/oder zwitterionischen und/oder amphoteren Tensiden, und/oder deren Mischungen,
c) Abdeckung der Keratinfasern mit einer Feuchtigkeitsbarriere,
d) Erhitzen der Keratinfasern auf eine Temperatur im Bereich von 50°C bis 230°C,
e) Entfernung der Feuchtigkeitsbarriere von Keratinfasern,
f) Lösen der Spannung von Keratinfasern,
g) gegebenenfalls Abspülen der Keratinfasern,
wobei die Verfahrensschritte a), b) und f), g) in beliebiger Reihenfolge ausgeführt werden können, und
wobei die folgenden nicht reduzierenden und nicht oxidierenden Zusammensetzungen von Schritt b) ausgeschlossen sind:
| | Bsp. 3 | Ex. 4 | Bsp. 5 | Vgl. Ex. 5 |
|---|---|---|---|---|
| NaOH | 2.4 | 2.4 | 2.4 | 2.4 |
| Monoethanolamin | 3.6 | 3.6 | 3.6 | 3.6 |
| Taurin | 8.7 | 8.7 | 8.7 | 8.7 |
| Magnesiumglukonat | 0.125 | 0.125 | 0.125 | 0.125 |
| Pentylenglykol | 10 | 7.5 | 5 | - |
| Ethanol | - | 2.5 | 5 | - |
| Hydroxyethylcellulose | 1.5 | 1.5 | 1.5 | 1.5 |
| Polyquaternium-67 | 0.5 | 0.5 | 0.5 | 0.5 |
| Dimethicon | 0.2 | 0.2 | 0.2 | 0.2 |
| Mineralöl | 5 | 5 | 5 | 5 |
| Steareth-2 | 2 | 2 | 2 | 2 |
| Steareth-20 | 2 | 2 | 2 | 2 |
| Wasser | qsp 100 | qsp 100 | qsp 100 | qsp 100 |
| pH-Wert | 10.7 | 10.7 | 10.7 | 10.7 |

2. Verfahren nach Anspruch 1, **dadurch gekennzeichnet, dass** das Alkalisierungsmittel der alkalischen Zusammensetzung von Schritt b) ausgewählt ist aus Ammoniak und/oder dessen Salzen und/oder Guanidin und/oder dessen Salz(en) und/oder organischen Alkyl- und/oder Alkanolaminen gemäß der allgemeinen Struktur 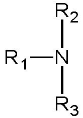 worin R1, R2 und R3 unabhängig voneinander ausgewählt sind aus H, linearem C1-C6-Alkyl, das mit einer Hydroxylgruppe substituiert sein kann, oder verzweigtem C3-C12-Alkyl oder Alkanol, wobei mindestens eines von R1, R2 oder R3 von H verschieden ist, und/oder deren Salze und/oder deren Gemische.

3. Verfahren nach Anspruch 1 und/oder 2, **dadurch gekennzeichnet, dass** das Alkalisierungsmittel der alkalischen Zusammensetzung von Schritt b) ausgewählt ist aus Aminomethylpropanol und/oder Monoethanolamin und/oder Tris-(hydroxymethyl)-aminomethan und/oder Diethanolamin und/oder deren Salzen.

4. Verfahren nach einem der vorhergehenden Ansprüche, **dadurch gekennzeichnet, dass** das Alkalisierungsmittel der alkalischen Zusammensetzung von Schritt b) Ammoniak und/oder seine Salze ist.

5. Verfahren nach einem der vorhergehenden Ansprüche, **dadurch gekennzeichnet, daß** die Gesamtkonzentration des Alkalisierungsmittels der alkalischen Zusammensetzung von Schritt b) im Bereich von 0,1 bis 10 Gew.-%, vorzugsweise im Bereich von 0,25 bis 7,5 Gew.-%, besonders bevorzugt im Bereich von 0,5 bis 5 Gew.-%, bezogen auf das Gesamtgewicht der Zusammensetzung von Schritt b), liegt.

6. Verfahren nach einem der vorhergehenden Ansprüche, **dadurch gekennzeichnet, dass** die anionischen Tenside der alkalischen Zusammensetzung von Schritt b) ausgewählt sind aus ethoxylierten oder nicht-ethoxylierten Alkylethersulfat-Tensiden, ethoxylierten oder nicht-ethoxylierten Alkylsulfaten, ethoxylierten und/oder nicht-ethoxylierten Alkylethercarboxylaten und/oder deren Mischungen.

7. Verfahren nach einem der vorhergehenden Ansprüche, **dadurch gekennzeichnet, dass** die nichtionischen Tenside der alkalischen Zusammensetzung von Schritt b) ausgewählt sind aus Alkylpolyglycosid(en), ethoxylierten und/oder propoxylierten Fettalkoholen, ethoxylierten und/oder propoxylierten Triglyceriden, ethoxylierten und/oder propoxylierten Fettalkoholen, ethoxylierten oder propoxylierten Fettsäureestern, N-Alkylpolyhydroxyalkylamiden und/oder deren Mischungen.

8. Verfahren nach einem der vorhergehenden Ansprüche, **dadurch gekennzeichnet, dass** die Gesamtkonzentration der Tenside in der alkalischen Zusammensetzung von Schritt b) im Bereich von 0,1 bis 10 Gew.-%, vorzugsweise 0,5 bis 8 Gew.-%, noch bevorzugter 0,75 bis 5 Gew.-%, berechnet auf die Gesamtmenge der Zusammensetzung von Schritt b), liegt.

9. Verfahren nach einem der vorangehenden Ansprüche, **dadurch gekennzeichnet, dass** die alkalische Zusammensetzung von Schritt b) eine Verbindung der allgemeinen Struktur 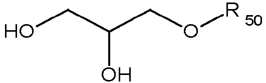 worin R50 eine lineare oder verzweigte Alkylkette mit einer Gesamtkohlenstoffzahl von C3 bis C12, vorzugsweise C3 bis C8, ist, besonders bevorzugt ist R50 eine verzweigte Alkylkette mit einer Gesamtkohlenstoffzahl von C8, vorzugsweise ist es Ethylhexylglycerin.

10. Verfahren nach einem der vorhergehenden Ansprüche, **dadurch gekennzeichnet, dass** Keratinfasern gemäß Schritt d) im Bereich von 80°C bis 180°C, vorzugsweise 85°C bis 140°C, noch bevorzugter 90°C bis 120°C, erhitzt werden.

11. Verfahren nach einem der vorhergehenden Ansprüche, **dadurch gekennzeichnet, dass** die Erhitzungszeit von Schritt d) im Bereich von 2 min bis 45 min, vorzugsweise im Bereich von 5 min bis 30 min, besonders bevorzugt im Bereich von 5 min bis 20 min liegt.

12. Verfahren nach einem der vorhergehenden Ansprüche, **dadurch gekennzeichnet, dass** für den Verfahrensschritt a) das Haar auf einem Lockenwickler oder einer Rolle mit Heizvorrichtung unter mechanischen Zug gesetzt wird.

13. Verfahren nach einem der vorhergehenden Ansprüche, **dadurch gekennzeichnet, dass** die Feuchtigkeitsbarriere von Schritt c) eine wasserdampfundurchlässige Folie oder Hülle oder ein Gehäuse aus einem wasserdampfundurchlässigen Material ist, mit der Maßgabe, dass die gewählten Materialien für die Feuchtigkeitsbarriere bis zu der gewählten Prozesstemperatur hitzebeständig sind.

14. Kit, enthaltend in einem separat verpackten Behälter eine Zusammensetzung, wie sie in den Ansprüchen 1 bis 9 für Schritt b) definiert ist, und eine Feuchtigkeitssperre, wie sie in Anspruch 13 für Schritt b) definiert ist.

15. Schaumspender, der eine nicht reduzierende, nicht oxidierende alkalische Zusammensetzung mit einem pH-Wert im Bereich von 7 bis 12, vorzugsweise 8 bis 11, umfasst:
i) einem oder mehreren Alkalisierungsmittel(n),
ii) ein oder mehrere schäumende(s) Tensid(e), ausgewählt aus nichtionischen und/oder anionischen und/oder zwitterionischen und/oder amphoteren Tensiden, und/oder deren Mischungen, und
iii) ein oder mehrere Treibgas(e),
wobei die Gesamtkonzentration der Treibmittel im Bereich von 30 bis 90 Gew.-%, vorzugsweise im Bereich von 45 bis 85 Gew.-%, besonders bevorzugt im Bereich von 60 bis 80 Gew.-%, bezogen auf die Gesamtmenge der Zusammensetzung, liegt.

## Revendications

1. Procédé de remodelage des fibres kératiniques, de préférence des fibres kératiniques humaines, et plus particulièrement des cheveux humains, **caractérisé par le fait qu'**il comprend les étapes suivantes :
a) la mise sous tension mécanique des fibres de kératine,
b) appliquer aux fibres kératiniques une composition alcaline non réductrice et non oxydante dont le pH est compris entre 7 et 12, de préférence entre 8 et 11, comprenant
i) un ou plusieurs agents alcalinisants,
ii) un ou plusieurs agents de surface moussants choisis parmi les agents de surface non ioniques et/ou anioniques et/ou zwitterioniques et/ou amphotères, et/ou leurs mélanges,
c) recouvrir les fibres de kératine d'une barrière contre l'humidité,
d) chauffer les fibres de kératine à une température comprise entre 50°C et 230°C,
e) éliminer la barrière d'humidité des fibres kératiniques,
f) relâcher la tension des fibres kératiniques,
g) rincer éventuellement les fibres kératiniques,
dans lequel les étapes a), b), et f), g) peuvent être exécutées dans n'importe quel ordre, et
les compositions non réductrices et non oxydantes suivantes étant exclues de l'étape b) :
| | Ex. 3 | Ex. 4 | Ex. 5 | Comp. Ex. 5 |
|---|---|---|---|---|
| NaOH | 2.4 | 2.4 | 2.4 | 2.4 |
| Monoéthanolamine | 3.6 | 3.6 | 3.6 | 3.6 |
| Taurine | 8.7 | 8.7 | 8.7 | 8.7 |
| Gluconate de magnésium | 0.125 | 0.125 | 0.125 | 0.125 |
| Pentylène glycol | 10 | 7.5 | 5 | - |
| Ethanol | - | 2.5 | 5 | - |
| Hydroxyéthylcellulose | 1.5 | 1.5 | 1.5 | 1.5 |
| Polyquaternium-67 | 0.5 | 0.5 | 0.5 | 0.5 |
| Diméthicone | 0.2 | 0.2 | 0.2 | 0.2 |
| Huile minérale | 5 | 5 | 5 | 5 |
| Steareth-2 | 2 | 2 | 2 | 2 |
| Steareth-20 | 2 | 2 | 2 | 2 |
| L'eau | qsp 100 | qsp 100 | qsp 100 | qsp 100 |
| pH | 10.7 | 10.7 | 10.7 | 10.7 |

2. Procédé selon la revendication 1 **caractérisé par le fait que** l'agent alcalin de la composition alcaline de l'étape b) est choisi parmi l'ammoniaque et/ou ses sels et/ou la guanidine et/ou son (ses) sel(s) et/ou les alkylamines et/ou les alcanolamines organiques selon la structure générale suivante 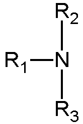 dans lequel R1, R2 et R3 sont indépendamment choisis parmi H, alkyle linéaire en C1-C6 pouvant être substitué par un groupe hydroxyle, ou alkyle ramifié en C3-C12 ou alcanol, dans lequel au moins un des R1, R2 ou R3 est différent de H, et/ou leurs sels, et/ou leurs mélanges.

3. Procédé selon la revendication 1 et/ou 2, **caractérisé par le fait que** l'agent alcalinisant de la composition alcaline de l'étape b) est choisi parmi l'aminométhylpropanol et/ou la monoéthanolamine, et/ou le tris-(hydroxyméthyl)-aminométhane, et/ou la diéthanolamine, et/ou leurs sels.

4. Procédé selon l'une quelconque des revendications précédentes, **caractérisé par le fait que** l'agent alcalinisant de la composition alcaline de l'étape b) est l'ammoniaque et/ou ses sels.

5. Procédé selon l'une des revendications précédentes se **caractérise par le fait que** la concentration totale d'agent alcalin de la composition alcaline de l'étape b) est comprise entre 0,1 % et 10 % en poids, de préférence entre 0,25 % et 7,5 % en poids, plus préférentiellement entre 0,5 % et 5 % en poids, par rapport au poids total de la composition de l'étape b).

6. Procédé selon l'une quelconque des revendications précédentes, **caractérisé par le fait que** les tensioactifs anioniques de la composition alcaline de l'étape b) sont choisis parmi les tensioactifs de type alkyl éther sulfate éthoxylés ou non éthoxylés, les alkyl sulfates éthoxylés ou non éthoxylés, les alkyl éther carboxylates éthoxylés et/ou non éthoxylés, et/ou leurs mélanges.

7. Procédé selon l'une quelconque des revendications précédentes, **caractérisé par le fait que** les tensioactifs non ioniques de la composition alcaline de l'étape b) sont choisis parmi les polyglycosides d'alkyle, les alcools gras éthoxylés et/ou propoxylés, les triglycérides éthoxylés et/ou propoxylés, les alcools gras éthoxylés et/ou propoxylés, les esters d'acide gras éthoxylés ou propoxylés, les N-alkylpolyhydroxyalkylamides, et/ou leurs mélanges.

8. Procédé selon l'une quelconque des revendications précédentes, **caractérisé par le fait que** la concentration totale de tensioactifs dans la composition alcaline de l'étape b) est comprise entre 0,1 % et 10 % en poids, de préférence entre 0,5 % et 8 % en poids, plus préférentiellement entre 0,75 % et 5 % en poids, calculée par rapport au total de la composition de l'étape b).

9. Procédé selon l'une quelconque des revendications précédentes **caractérisé en ce que** la composition alcaline de l'étape b) comprend un composé selon la structure générale 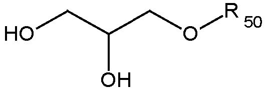 dans laquelle R50 est une chaîne alkyle linéaire ou ramifiée avec un nombre total de carbone de C3 à C12, de préférence de C3 à C8, plus préférentiellement R50 est une chaîne alkyle ramifiée avec un nombre total de carbone de C8, de préférence c'est l'éthylhexylglycérine.

10. Procédé selon l'une quelconque des revendications précédentes est **caractérisé par le fait que** les fibres de kératine sont chauffées selon l'étape d) entre 80°C et 180°C, de préférence entre 85°C et 140°C, plus préférentiellement entre 90°C et 120°C.

11. Procédé selon l'une des revendications précédentes est **caractérisé par le fait que** le temps de chauffage de l'étape d) est compris entre 2 min et 45 min, de préférence entre 5 min et 30 min, plus préférentiellement entre 5 min et 20 min.

12. Procédé selon l'une quelconque des revendications précédentes est **caractérisé par le fait que**, pour l'étape a), les cheveux sont soumis à une tension mécanique sur un bigoudi ou un rouleau doté d'un dispositif de chauffage.

13. Procédé selon l'une des revendications précédentes est **caractérisé par le fait que** la barrière contre l'humidité de l'étape c) est une feuille ou un emballage imperméable à la vapeur d'eau, ou un boîtier fait d'un matériau imperméable à la vapeur d'eau, à condition que les matériaux sélectionnés pour la barrière contre l'humidité soient résistants à la chaleur jusqu'à la température de traitement sélectionnée.

14. Kit de pièces comprenant dans un récipient emballé séparément une composition telle que définie pour l'étape b) dans les revendications 1 à 9, et une barrière contre l'humidité telle que définie dans la revendication 13 pour l'étape b).

15. Distributeur de mousse comprenant une composition alcaline non réductrice et non oxydante ayant un pH compris entre 7 et 12, de préférence entre 8 et 11, comprenant :
i) un ou plusieurs agents alcalinisants,
ii) un ou plusieurs agents de surface moussants choisis parmi les agents de surface non ioniques et/ou anioniques et/ou zwitterioniques et/ou amphotères, et/ou leurs mélanges, et
iii) un ou plusieurs agents propulseurs,
dans lequel la concentration totale des agents propulseurs est comprise entre 30 % et 90 % en poids, de préférence entre 45 % et 85 % en poids, plus préférentiellement entre 60 % et 80 % en poids, calculée par rapport au total de la composition.
